(19) Europäisches Patentamt
European Patent Office
Office européen des brevets

(11) EP 1 732 488 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**07.05.2014   Patentblatt 2014/19**

(45) Hinweis auf die Patenterteilung:
**29.12.2010   Patentblatt 2010/52**

(21) Anmeldenummer: 05729343.3

(22) Anmeldetag: **31.03.2005**

(51) Int Cl.:
***A61F 13/532*** *(2006.01)*

(86) Internationale Anmeldenummer:
**PCT/EP2005/003397**

(87) Internationale Veröffentlichungsnummer:
**WO 2005/094745 (13.10.2005 Gazette 2005/41)**

(54) **HYGIENEARTIKEL ZUM EINMALIGEN GEBRAUCH**

DISPOSABLE HYGIENE ARTICLE

ARTICLE D'HYGIENE A USAGE UNIQUE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **03.04.2004   DE 102004016552**

(43) Veröffentlichungstag der Anmeldung:
**20.12.2006   Patentblatt 2006/51**

(73) Patentinhaber: **Paul Hartmann Aktiengesellschaft**
**89522 Heidenheim (DE)**

(72) Erfinder: **WENDELSTORF, Carsten**
**56068 Koblenz (DE)**

(74) Vertreter: **DREISS Patentanwälte PartG mbB**
**Patentanwälte**
**Postfach 10 37 62**
**70032 Stuttgart (DE)**

(56) Entgegenhaltungen:
| | |
|---|---|
| **EP-A- 1 101 477** | **EP-B- 1 006 970** |
| **WO-A-03/055431** | **WO-A-2004/105668** |
| **US-A- 4 449 979** | **US-A- 5 451 442** |
| **US-A1- 2002 052 587** | **US-A1- 2002 052 587** |
| **US-B1- 6 498 283** | |

EP 1 732 488 B2

**Beschreibung**

Die Erfindung betrifft einen Hygieneartikel zum einmaligen Gebrauch, mit einer der Speicherung von Körperflüssigkeiten dienenden Saugkörperkomponente, die auch superabsorbierende Materialien enthalten kann.

Ein Hygieneartikel mit in einem Bereich in Querrichtung zu den Seitenrändern hin zunehmendem Flächengewicht eines absorbierenden Materials ist in der nicht vorveröffentlichten Patentanmeldung DE 103 26 022.6 der Anmelderin beschrieben.

Bei Hygieneartikeln der eingangs genannten Art, also insbesondere Babywindeln, Inkontinenzwindeln und -hosen sowie absorbierenden Einlagen, aber auch Monatsbinden und Slipeinlagen, erweist sich der Schrittbereich insoweit als problematisch, als dort einerseits eine hinreichende Absorptionskapazität zur Verfügung gestellt werden soll, andererseits aber der zur Verfügung stehende Platz zwischen den Beinen des Benutzers beschränkt ist. Wird im Schrittbereich zu viel voluminöses Saugkörpermaterial vorgesehen, so führt dies zu einem unangenehmen Traggefühl, und es kommt zu Verwindungen und Aufwerfungen, welche die Funktion des Hygieneartikels beeinträchtigen. Es wurde deshalb bereits der Vorschlag unterbreitet, im Schrittbereich ein Maximum an superabsorbierenden partikelförmigen Materialien vorzusehen, die eine hohe dauerhafte Speicherkapazität bei sehr geringem Ausgangsvolumen im trockenen Zustand aufweisen, was für sich genommen bekannt ist und daher keiner weitergehenden Erläuterung bedarf. Die oben genannte DE 103 26 022.6 lehrt auch bereits, Saugkapazität vom Schrittbereich weg in Richtung auf den Vorderbereich und den Rückenbereich zu verlagern.

Der vorliegenden Erfindung liegt die Aufgabe zugrunde, einen Hygieneartikel zu schaffen, der eine optimale Verteilung von Absorptionskapazität aufweist und dem Benutzer dennoch ein angenehmes Traggefühl vermittelt.

Diese Aufgabe wird durch einen Hygieneartikel mit den Merkmalen des Anspruchs 1 gelöst.

Es wird erfindungsgemäß vorgeschlagen, dass eine Saugkörperkomponente in wenigstens einem ersten Bereich in Querrichtung ein zunehmendes Flächengewicht und damit eine zunehmende Speicherkapazität eines absorbierenden Materials aufweist. Ferner wird vorgeschlagen, die Saugkörperkomponente so auszubilden, dass sie trotz einer gegebenenfalls vorgesehenen Taillierung im Schrittbereich (Sanduhrform) zumindest über eine gewisse Abschnittslänge in Längsrichtung betrachtet eine im Wesentlichen konstante Speicherkapazität aufweist. Es wurde nämlich erfindungsgemäß festgestellt, dass eine in Längsrichtung des Hygieneartikels eher gleichmäßige Verteilung der Speicherkapazität, und zwar gerade bei taillierten Saugkörperkomponenten mit einem angenehmen Traggefühl einhergeht, was wiederum die Betriebseigenschaften des Hygieneartikels als Ganzes positiv beeinflusst, da der Hygieneartikel und dessen Saugkörperkomponenten weniger stark deformiert oder verdrängt werden. Weiter erfindungsgemäß weist die Saugkörperkomponente einen zweiten Bereich mit ausgehend vom Rücken- und Vorderbereich des Hygieneartikels zum Schrittbereich hin zunehmendem Flächengewicht eines absorbierenden Materials dieser Saugkörperkomponente auf. Hierdurch lässt sich dem Umstand Rechnung tragen, dass bei taillierten Saugkörpern bei Betrachtung aneinandergrenzender Längsabschnitte aufgrund der Taillierung eine geringere Fläche zur Verfügung steht, was dann bei gleichbleibendem Flächengewicht (von Abschnitt zu Abschnitt) zu einer Reduzierung der Speicherkapazität im taillierten Bereich führen würde. Dadurch, dass in Richtung auf den insbesondere tailliert ausgebildeten Schrittbereich hin in dem zweiten Bereich ein zunehmendes Flächengewicht eines absorbierenden Materials der Saugkörperkomponente vorgesehen wird, lässt sich die Speicherkapazität in Längsrichtung im Wesentlichen konstant halten.

Wenn vorstehend von einer Saugkörperkomponente die Rede ist, so kann hierunter ein Saugkörper eines in Rede stehenden Hygieneartikels in seiner Gesamtheit oder aber eine Schicht eines mehrschichtigen Saugkörpers oder ein dreidimensionaler Bestandteil desselben verstanden werden. Wenn vorstehend von Speicherkapazität die Rede ist, so wird hierunter das Vermögen zur dauerhaften Speicherung von Flüssigkeiten in Saugkörpermaterialien verstanden, welches Vermögen, wie nachfolgend im Einzelnen erörtert wird, durch das Retentionsvermögen in einem Zentrifugentest bestimmt und definiert ist.

Die vorstehend erwähnte Abschnittslänge von 40 bis 90% der Länge der betrachteten Saugkörperkomponente wird durch Vergleich der Speicherkapazität von Längsabschnitten des Hygieneartikels oder des Saugkörpers bzw. der Saugkörperkomponente bestimmt. Hierbei wird der Saugkörper oder die Saugkörperkomponente in auf eine ebene Unterlage ausgelegtem Zustand entlang der Längserstreckung in Längsabschnitte unterteilt, insbesondere einer Länge von 5 bis 40 mm, zweckmäßigerweise in Längsabschnitte von etwa 20 mm Länge. Es wird dann experimentell und/oder rechnerisch die Speicherkapazität eines jeweiligen Längsabschnitts des Saugkörpers oder der Saugkörperkomponente bestimmt, um sie miteinander vergleichen zu können.

Zur Bestimmung der Speicherkapazität wird auf einen Zentrifugentest zurückgegriffen, in dem die Flüssigkeitsretention absorbierender Materialien bei einer definierten Beschleunigung von 276 g ($g$ = 9,81 m/sec$^2$) nach einer Schleuderzeit von 4 min ermittelt wird. Dieser Zentrifugentest lässt sich heranziehen, um die Speicherkapazität an sich beliebiger absorbierender Strukturen oder auch Bestandteilen absorbierender Strukturen, wie Fasern einer bestimmten Art, z. B. gefluffte Zellulosefasern oder intravernetzte Zellulosefasern oder superabsorbierende Materialien, zu ermitteln. Hierfür wird unter Verwendung einer Präzisionswaage die Ausgangsmasse einer Probe auf Zehntelgramm bestimmt. Die Probe wird danach 20 min in einer 99,5% NaCl-Lösung (nicht gefärbt oder vergällt, in demineralisiertem Wasser) getaucht.

Danach werden die Proben gegen die Trommelwand einer Zentrifuge gelegt (sofern flüssigkeitsundurchlässige Materialien (wie Backsheetfolien) vorhanden sind, werden diese nach radial innen orientiert, um den Flüssigkeitsaustritt nicht zu behindern). Die Proben werden dann 4 min lang einem Schleudervorgang bei 276 g unterzogen. Danach werden sie erneut gewogen. Die Flüssigkeitsretention und damit die Speicherkapazität kann in Gramm als Differenz der bestimmten Massen nach dem Schleudertest ($M_{nass}$) und vor dem Schleudertest ($M_{trocken}$) bestimmt werden:

$$\text{Flüssigkeitsretention} = M_{nass} - M_{trocken} \text{ (in g)}.$$

Das Ergebnis kann auch in Bezug auf die Masse (in g/g) wie folgt angegeben werden:

$$\text{Flüssigkeitsretention (relativ)} = \frac{M_{nass} - M_{trocken}}{M_{trocken}}$$

Das Ergebnis ist auf ganze Zahlen zu runden und in g bzw. in g/g anzugeben. Die Prüfanzahl soll jeweils mindestens 6 betragen, wobei ein Durchschnittswert x, sowie $x_{min}$ und $x_{max}$ sowie vorzugsweise die Standardabweichung S angegeben werden sollen.

Es erweist sich insbesondere als vorteilhaft, wenn für die bei einem Saugkörper verwandten absorbierenden Materialien die Speicherkapazität in der vorstehend genannten Weise bestimmt wird. Es lässt sich dann über Flächengewichtsbetrachtungen, also über Berücksichtigung oder Zugrundelegung des Flächengewichts eines jeweiligen absorbierenden Materials in einem Saugkörperbereich die Speicherkapazität dieses Saugkörperbereichs bestimmen. Auf diese Weise lassen sich an sich beliebige Profile der Speicherkapazität eines Saugkörpers oder einer Saugkörperkomponente über an sich beliebige Bereiche oder Richtungen angeben.

In weiterer Präzisierung des Erfindungsgedankens wird vorgeschlagen, dass sich der Abschnitt konstanter Speicherkapazität in Längsrichtung über 40 bis 70 % und weiter insbesondere über 45 bis 60 % der Länge der betrachteten Saugkörperkomponente erstreckt. Dieser Abschnitt wird sich dann in vorteilhafter Weise im Wesentlichen im Schrittbereich des Hygieneartikels, also im Bereich zwischen den Beinen des Benutzers befinden.

Wenn Speicherkapazitäten von Saugkörperkomponenten in aneinander angrenzenden Längsabschnitten eines Hygieneartikels oder Saugkörpers von 10 mm Länge miteinander verglichen werden, so wird eine Abweichung von bis zu ± 15% noch als im anspruchsgemäßen Sinn im Wesentlichen konstant angesehen. Vorzugsweise beträgt die Abweichung weniger als 10%.

In weiterer Präzisierung des Erfindungsgedankens weist der erste Bereich zunehmenden Flächengewichts eines absorbierenden Materials einer Saugkörperkomponente eine Flächengewichtszunahme in Querrichtung um 30 bis 200 %, insbesondere um 30 bis 150 % und weiter insbesondere um 50 bis 120 % auf.

Der zweite Bereich zunehmenden Flächengewichts eines absorbierenden Materials der Saugkörperkomponente weist eine Flächengewichtszunahme in Längsrichtung um 50 bis 500 %, insbesondere um 50 bis 400 % und weiter insbesondere um 100 bis 350 % auf.

Des Weiteren erweist es sich als vorteilhaft, wenn das maximale Flächengewicht des zweiten Bereichs zunehmenden Flächengewichts größer ist als das maximale Flächengewicht des ersten Bereichs zunehmenden Flächengewichts. Hierdurch lässt sich auch einer starken Taillierung im erfindungsgemäßen Sinn Rechnung tragen.

Der erste Bereich zunehmenden Flächengewichts in Querrichtung muss nicht notwendigerweise im Schrittbereich eines Hygieneartikels zu liegen kommen. Angesichts einer häufig anzutreffenden und gewünschten Taillierung des Saugkörpers oder einer Saugkörperkomponente im Schrittbereich erweist es sich als vorteilhaft, wenn dieser erste Bereich zunehmenden Flächengewichts von einem Zentrum des Schrittbereichs des Hygieneartikels beabstandet ist. Er kann insbesondere außerhalb des Schrittbereichs in einem Vorderbereich und/oder in einem Rückenbereich des Hygieneartikels liegen.

Üblicherweise wird der Schrittbereich als derjenige Bereich eines Hygieneartikels angesehen, der im Gebrauch zwischen den Beinen eines Benutzers zu liegen kommt. Das Zentrum des Schrittbereichs eines Hygieneartikels lässt sich bestimmen, indem bei einem aufrecht stehenden Benutzer oder einem langgestreckt auf eine ebene Unterlage gelegten Baby ein elastischer Faden oder ein Gummiband achtförmig um die Beine herumgelegt wird, so dass ein Kreuzungspunkt des Fadens oder Bands zwischen den Beinen entsteht. Dieser Kreuzungspunkt wird als Zentrum des Schrittbereichs des Hygieneartikels definiert, wenn dieser bestimmungsgemäß getragen wird. Eine Abgrenzung von Schrittbereich und Vorderbereich bzw. Rückenbereich des Hygieneartikels ist naturgemäß zumindest exakt schwer definierbar. Zum Zwecke einer dennoch quantitativen Abgrenzung wird der Schrittbereich als derjenige Bereich des Hygieneartikels definiert, der sich ausgehend vom Zentrum des Schrittbereichs bis 25 % der Gesamtlänge des Saugkörpers in den Vorbereich und bis 25 % der Gesamtlänge des Saugkörpers in den Rückenbereich des Hygieneartikels erstreckt und dort jeweils übergeht in den Vorderbereich bzw. den Rückenbereich.

In Weiterbildung des zuletzt genannten Erfindungsgedankens erweist es sich als vorteilhaft, wenn zwei erste Bereiche zunehmenden Flächengewichts in Querrichtung vorgesehen sind, die vom Zentrum des Schrittbereichs des Hygieneartikels in Längsrichtung beabstandet sind, also insbesondere im Vorderbereich bzw. Rückenbereich des Hygieneartikels liegen.

Es erweist sich auch als vorteilhaft, wenn der erste Bereich oder eine Linie maximalen Flächengewichts des ersten Bereichs beidseits des Hygieneartikels über wenigstens 15 % der Länge der Saugkörperkomponente in Längsrichtung erstreckt sind. In Draufsicht auf den Saugkörper oder die Saugkörperkomponente können sich solchenfalls beidseits streifenförmige erste Bereiche in Längsrichtung erstrecken. Diese streifenförmigen Bereiche können sich des Weiteren vorzugsweise entlang von Seitenrandbereichen des Hygieneartikels erstrecken. Sie bilden daher in gewisser Weise Flanken oder einen flankierenden Auslaufschutz.

In ganz besonders vorteilhafter Weise sind ein erster Bereich zunehmenden Flächengewichts in Querrichtung und ein zweiter Bereich zunehmenden Flächengewichts in Längsrichtung unmittelbar angrenzend aneinander oder überlappend miteinander vorgesehen. Dies ist dann der Fall, wenn anhand der Saugkörpertopographie die betrachteten Bereiche quasi ineinander übergehen.

Es wurde bereits darauf hingewiesen, dass sich die Erfindung als besonders vorteilhaft erweist, wenn sich die Breite der Saugkörperkomponente ausgehend vom Rücken- und/oder Vorderbereich des Hygieneartikels zum Schrittbereich hin verringert, wenn die betrachtete Saugkörperkomponente und insbesondere der gesamte Saugkörper tailliert oder sanduhrförmig oder nur T-förmig ausgebildet ist.

Die erfindungsgemäß geforderte Zunahme eines absorbierenden Materials einer Saugkörperkomponente kann in besonders einfacher Weise durch eine Anhäufung des betreffenden Materials realisiert werden. Hierzu können entsprechende Formgestaltungen als Negativformen verwendet werden, die dann bei der Herstellung des Saugkörpers bzw. der Saugkörperkomponente zu einer dem zunehmenden Flächengewicht entsprechenden Saugkörpertopographie führen. Ausgehend von einer solchen im Wesentlichen mit gleichförmiger Dichte aufgebauten Saugkörpertopographie kann es sich als vorteilhaft erweisen, wenn der Saugkörper oder die betrachtete Saugkörperkomponente im Anschluss an die Herstellung der Topographie auf eine im Wesentlichen einheitliche Dicke verpresst wird. Es erweist sich dann als vorteilhaft, dass Bereiche zunehmenden Flächengewichts auch Bereiche zunehmender Dichte bilden.

Eine erfindungsgemäße Saugkörperkomponente kann wenigstens zwei Saugkörperschichten umfassen, wobei eine der Saugkörperschichten ein im Wesentlichen einheitliches Flächengewicht aufweist, also in Längsrichtung und Querrichtung ein konstantes gleichbleibendes Flächengewicht aufweist. Bei dieser Schicht kann es sich insbesondere um eine Basis- oder Grundmatte oder eine körperzugewandte Verteilerschicht handeln.

Des Weiteren erweist es sich als vorteilhaft, wenn die Saugkörperkomponente eine vernetzte Zellulosefasern umfassende Saugkörperschicht aufweist. Vernetzte Zellulosefasern erweisen sich insbesondere im eingenässten Zustand als bauschelastisch; sie bewahren ein zur raschen Aufnahme von Flüssigkeit erforderliches großes Porenvolumen und neigen nicht zum "wet collapse", weshalb sie gern als körperzugewandt angeordnete Flüssigkeitsaufnahme und -verteilerschichten bei absorbierenden Strukturen für Hygieneartikel verwendet werden.

In noch weitergehender Ausbildung der Erfindung erweist es sich als vorteilhaft, wenn der Hygieneartikel seitliche Auslaufsperren bildende, im Wesentlichen in einer Längsrichtung verlaufende und zumindest bereichsweise aufstehende Cuffelemente, die zumindest entlang einer Cuffsockellinie an der körperzugewandten Seite des Artikels festgelegt sind, aufweist. Unter "Cuffelementen" werden an sich bekannte Barriere- oder Seitenauslaufschutzelemente verstanden, die zumeist eine elastifizierende Komponente umfassen, welche bewirkt, dass sich die Cuffelemente im Benutzungszustand emporheben und gegen die Hautoberfläche des Benutzers anlegen.

Es erweist sich ferner als vorteilhaft, wenn diese Cuffelemente mit variierendem Abstand (in Querrichtung) der Cuffsockellinien voneinander verlaufen. In noch weitergehender Ausbildung dieses Erfindungsgedankens weist der erste Bereich größeren Flächengewichts (in Querrichtung) zumindest einen Teilbereich auf, in dem der Abstand der Cuffsockellinien voneinander größer ist als außerhalb dieses Teilbereichs. Es wurde mit der Erfindung nämlich festgestellt dass die Cuffelemente mit variierendem Abstand ihrer Cuffsockellinien voneinander geführt werden können, so dass sie beispielsweise in einem Vorderbereich oder in einem Rückenbereich, also üblicherweise außerhalb eines Schrittbereichs des Hygieneartikels, weiter voneinander beabstandet sind und dass dies dann zu einer größeren Oberfläche führt, die zur Aufnahme von Flüssigkeit zur Verfügung steht. Wenn sich nämlich bei schwallartiger Flüssigkeitsbeaufschlagung die Flüssigkeit auf der Oberfläche des Hygieneartikels, also zwischen den aufstehenden Cuffelementen, verteilt, so erweist es sich für eine rasche Flüssigkeitsaufnahme als vorteilhaft, wenn die aufnehmende Fläche so groß wie möglich gewählt wird. Dies bedeutet aber, dass die Cuffelemente und die von ihnen gebildeten Taschen zur Aufnahme von Körperausscheidungen in die Nähe des Saugkörperrands gelangen, wo möglicherweise nicht ausreichend Absorptionskapazität zur Verfügung steht oder aufgrund der Wechselwirkung mit dem Rand des Saugkörpers eine den Anforderungen genügende Abdichtung oder Aufnahmekapazität nicht gewährleistet ist. Hinzu kommt, dass die Anbindung der Cuffelemente an die Materialien des Hygieneartikels die Gefahr kapillaren Flüssigkeitstransports zu den Rändern des Hygieneartikels hin in ungewollter Weise begünstigt, sofern dort keine Absorptionskapazität zur Verfügung steht. Mit der vorliegenden Erfindung wurde nun erkannt, dass durch die Erhöhung des Flächengewichts eines absorbierenden Materials der Saugkörperkomponente gerade dort, wo der Abstand der Cuffsockellinien voneinander größer ist als anderen Orts, eine bessere und weniger störanfällige Flüssigkeitsaufnahmecharakteristik des Hygieneartikels im Gebrauch erreicht werden kann.

Der vorstehend erwähnte Teilbereich ist vorzugsweise außerhalb eines mittleren Längsabschnitts des Hygieneartikels und im Abstand vom Zentrum des Schrittbereichs angeordnet. Er ist also vom Zentrum des Schrittbereichs weg in Richtung auf den Vorderbereich und/oder den Rückenbereich hin verlagert.

Die vorstehend erwähnten Cuffelemente sind in Längsrichtung vorzugsweise so geführt, dass ihr Abstand voneinander ein Maximum durchläuft. Es erweist sich als vorteilhaft, wenn dieses Maximum vollständig innerhalb des erwähnten Teilbereichs des ersten Bereichs größeren Flächengewichts (in Querrichtung) liegt.

Ferner erweist es sich als vorteilhaft, wenn das Maximum oder die Maxima des Abstands der Cuffsockellinien voneinander in einem Vorderbereich und/oder in einem Rückenbereich des Hygieneartikels angeordnet ist bzw. sind.

Die erwähnte Saugkörperkomponente umfasst vorteilhafterweise eine Mischung aus Fasern und partikelförmigen superabsorbierenden Materialien.

Weitere Merkmale, Vorteile und Einzelheiten des erfindungsgemäßen Hygieneartikels ergeben sich aus den beigefügten Patentansprüchen und aus der zeichnerischen Darstellung und nachfolgenden Beschreibung einer bevorzugten Ausführungsform der Erfindung.

In der Zeichnung zeigt:

Figur 1 in perspektivischer Darstellung die Negativform einer erfindungsgemäßen Saugkörpertopographie, die gebildet werden kann, indem die Saugkörpermaterialien in diese Negativform mit im Wesentlichen gleichförmiger Dichte ein- bzw. aufgegeben werden;

Figur 2 eine Draufsicht auf einen Saugkörper, der unter Verwendung der Negativform nach Figur 1 gebildet wurde;

Figuren 3,4 eine Darstellung der zur Verfügung stehenden Speicherkapazität in Längsabschnitten;

Figur 5 eine Draufsicht auf einen erfindungsgemäßen Hygieneartikel (schematisch) und

Figur 6 eine Schnittansicht des Hygieneartikels nach Figur 5.

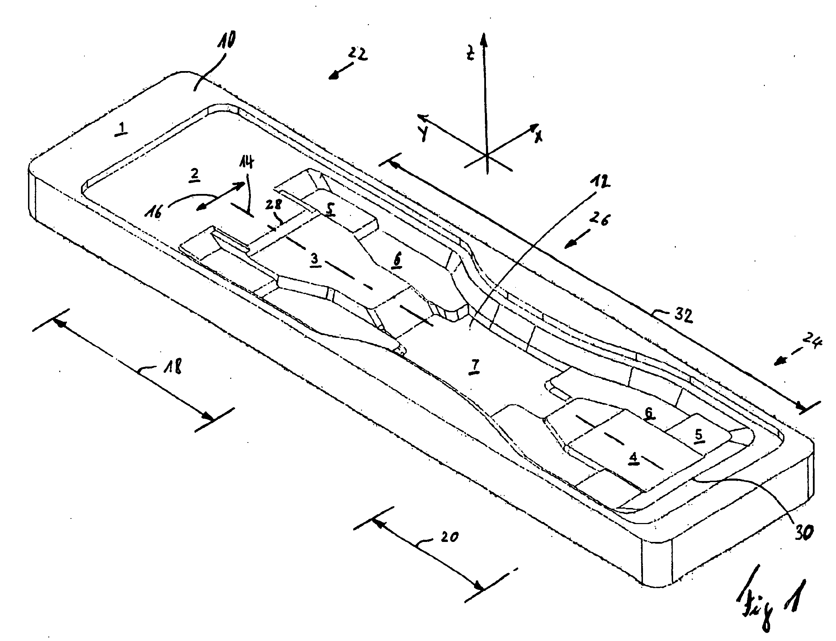

Die in Figur 1 mit 1 bezeichnete im Wesentlichen rechteckförmige Fläche der Form bezeichnet die Erstreckung einer Grundmatte von geflufften Zellulosefasern eines gleichförmigen Flächengewichts. Die mit den Bezugszeichen 2 bis 7 bezeichneten Gebiete der Form bilden ein dreidimensionales Volumen, als eine Topographie, für die erfindungsgemäße Ausbildung einer Saugkörperkomponente 12. Die erfindungsgemäße Saugkörperkomponente 12 kann beispielsweise dadurch erhalten werden, dass eine an sich homogene Mischung aus geflufften Zellulosefasern und superabsorbierenden partikelförmigen Materialien in die Gebiete 2 bis 7 der Form eingebracht werden. Je nach Tiefe der Form in Z-Richtung weist die Saugkörperkomponente 12 ein mehr oder weniger hohes Flächengewicht des die Saugkörperkomponente 12 bildenden absorbierenden Materials auf.

Betrachtet man beispielsweise die Gebiete 2 und 3 ausgehend von einer Längsmittellinie 14 (in Längsrichtung Y verlaufend) und deren Übergang zu den Gebieten 5 und 6 (in Querrichtung 16), so erkennt man, dass das Flächengewicht des absorbierenden Materials der Saugkörperkomponente 12 in Querrichtung zu den Seitenrändern der Saugkörperkomponente 12 hin beidseits zunimmt. In entsprechender Weise erkennt man dies ausgehend vom Gebiet 4 in Querrichtung 16 im Übergang zu den Gebieten 5, 6.

Die Saugkörperkomponente 12 weist daher zwei erste Bereiche 18, 20 mit zunehmendem Flächengewicht des absorbierenden Materials dieser Saugkörperkomponente in Querrichtung 16 zu den Seitenrändern hin auf.

Des Weiteren erkennt man, dass auch in Längsrichtung 14 ausgehend von einem Rückenbereich 22 und einem Vorderbereich 24 in Richtung auf einen Schrittbereich 26 das Flächengewicht des absorbierenden Materials der Saugkörperkomponente 12 zunimmt. Diese Zunahme des Flächengewichts der Saugkörperkomponente 12 beginnt bei Außerachtlassung der Grundmatte 10 an einer dem Schrittbereich 26 zugewandten Kante 28 des Gebiets 2 im Rückenbereich 22 und an einer Kante 30 des Gebiets 2 im Vorderbereich 24. Der Bereich zwischen den Kanten 28 und 30 wird daher als zweiter Bereich 32 zunehmenden Flächengewichts eines absorbierenden Materials der Saugkörperkomponente 12 in Längsrichtung 14 in Richtung auf den Schrittbereich 26 hin bezeichnet.

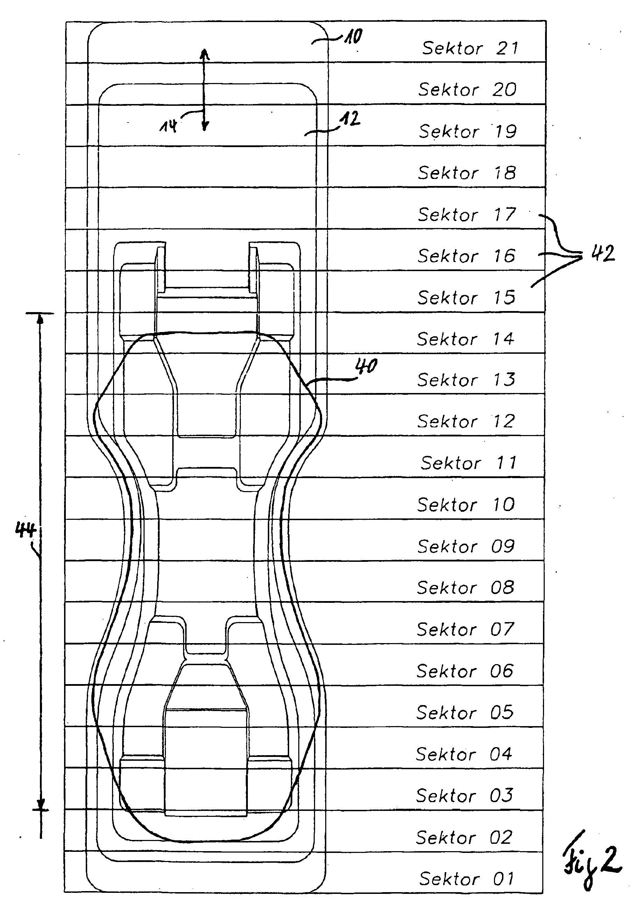

Figur 2 zeigt eine Draufsicht auf einen Saugkörper, der unter Verwendung der Form nach Figur 1 in der dort beschriebenen Weise hergestellt worden ist und die bereits erwähnte Grundmatte 10, die Saugkörperkomponente 12 und zusätzlich eine körperzugewandte Verteilerschicht 40, die sanduhrförmig ausgebildet ist und ungefähr die den Gebieten 3 bis 7 entsprechenden Teile der dreidimensionalen Struktur der Saugkörperkomponente 12 überdeckt. Der dargestellte Saugkörper umfasst also eine Grundmatte 10 gleichförmigen Flächengewichts und die im dargestellten Fall als dreidimensionale Topologie dargestellte Saugkörperkomponente 12 und eine Verteilerschicht 40 wiederum gleichförmigen Flächengewichts. Im lediglich beispielhaft dargestellten Fall besteht die Grundmatte 10 aus geflufften natürlichen Zellulosefasern, die Saugkörperkomponente 12 aus einer homogenen Mischung aus natürlichen geflufften Zellulosefasern und superabsorbierenden partikelförmigen Materialien (SAP) und die Verteilerschicht 40 aus intravernetzten Zellulosefasern.

In der Darstellung der Figur 2 erkennt man eine Unterteilung des Saugkörpers in 21 Längsabschnitte 42, also in Längsrichtung 14 aneinander angrenzende Abschnitte einer Länge von etwa 20 mm.

Die Saugkörperkomponente 12 wurde derart ausgebildet, dass die Speicherkapazität in Längsrichtung 14 über wenigstens 20 % der Länge der Saugkörperkomponente 12 im Wesentlichen konstant ist. Dabei werden die Längsabschnitte 42 (Sektoren 01 bis 21) herangezogen, und es wird deren Speicherkapazität, wie eingangs beschrieben, ermittelt.

Aus der nachfolgenden Tabelle sind die Flächengewichte einer beispielhaften und bevorzugten Saugkörperzusammensetzung in den jeweiligen Gebieten 1 bis 7 der Topologie nach Figur 1 angegeben. Es sind jeweils die Flächengewichte von natürlichen geflufften Zellulosefasern ("fluff") bzw. intravernetzten Zellulosefasern der Verteilerschicht 40 ("cf") und die Flächengewichte superabsorbierender Partikelmaterialien ("SAP") angegeben, und zwar jeweils in g/m$^2$.

| g/m$^2$ | Fluff | CF | SAP |
|---|---|---|---|
| Niveau 1 | 136 | 0 | 0 |
| Niveau 2 | 289 | 0 | 211 |
| Niveau 3 | 367 | 215 | 327 |
| Niveau 4 | 382 | 215 | 340 |
| Niveau 5 | 395 | 0 | 352 |
| Niveau 6 | 444 | 215 | 425 |
| Niveau 7 | 708 | 215 | 789 |

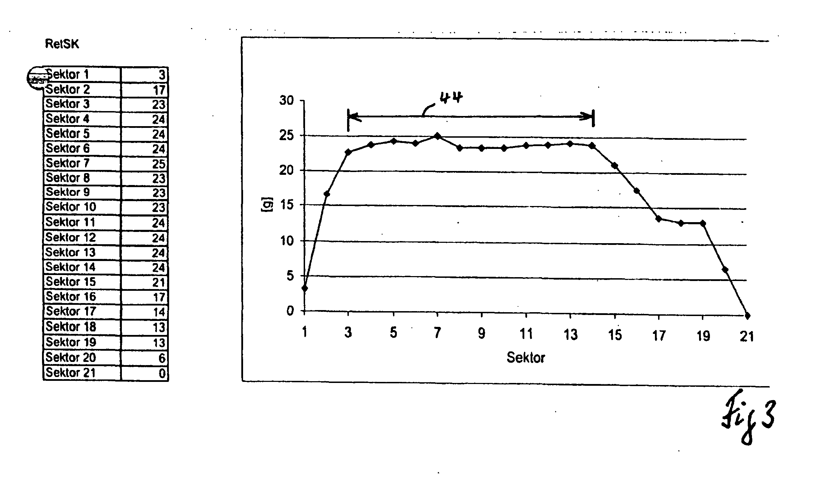

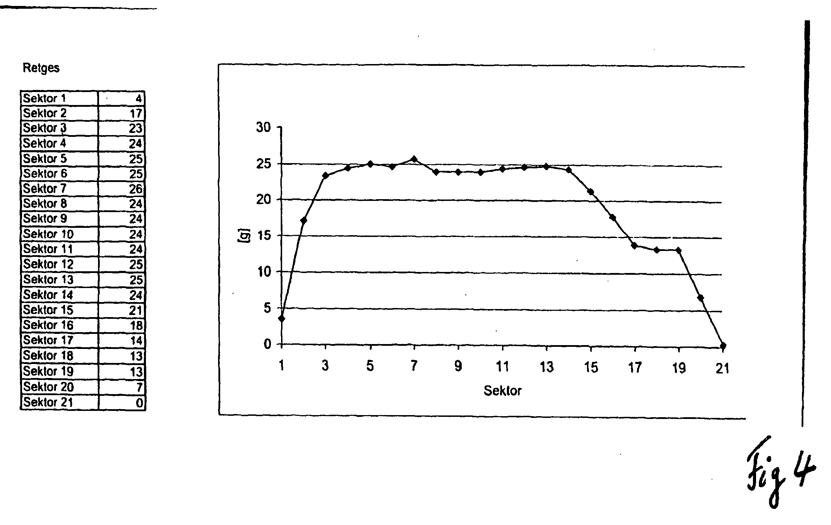

Die Anmelderin hat den Retentionswert der von ihr verwendeten superabsorbierenden Materialien und der von ihr verwendeten intravernetzten Zellulosefasern ("cf") und der natürlichen geflufften Zellulosefasern ("fluff") ermittelt. Für gefluffte natürliche Zellulosefasern und intravernetzte Zellulosefasern hat sich ein Retentionswert von 1 g/g und für die superabsorbierenden Materialien ein Retentionswert von 30 g/g nach der eingangs gegebenen Methode ergeben. Aus diesen Werten lässt sich nun unter Berücksichtigung der Flächen- bzw. Volumenanteile der jeweiligen Gebiete 1 bis 7 die in einem jeweiligen Längsabschnitt 42 (Sektoren 1 bis 21) zur Verfügung stehende Speicherkapazität rechnerisch ermitteln. Das Ergebnis ist in den Figuren 3 und 4 aufgezeigt.

Die Figuren 3 und 4 zeigen die Speicherkapazität (in g der speicherbaren Flüssigkeit), und zwar sowohl in Tabellenform als auch in Form einer Graphik. Bei den in Figur 4 und der dazugehörigen Tabelle geringfügig höheren Werten wurde die Grundmatte 10 und die Verteilerschicht 40 mitberücksichtigt.

Man erkennt über die Längsabschnitte 42, also in Längsrichtung 14 eine im Wesentlichen konstante Speicherkapazität in einem Abschnitt 44 der Saugkörperkomponente, welcher Abschnitt 44 die Sektoren 3 bis 14 der Längsabschnitte 42 umfasst.

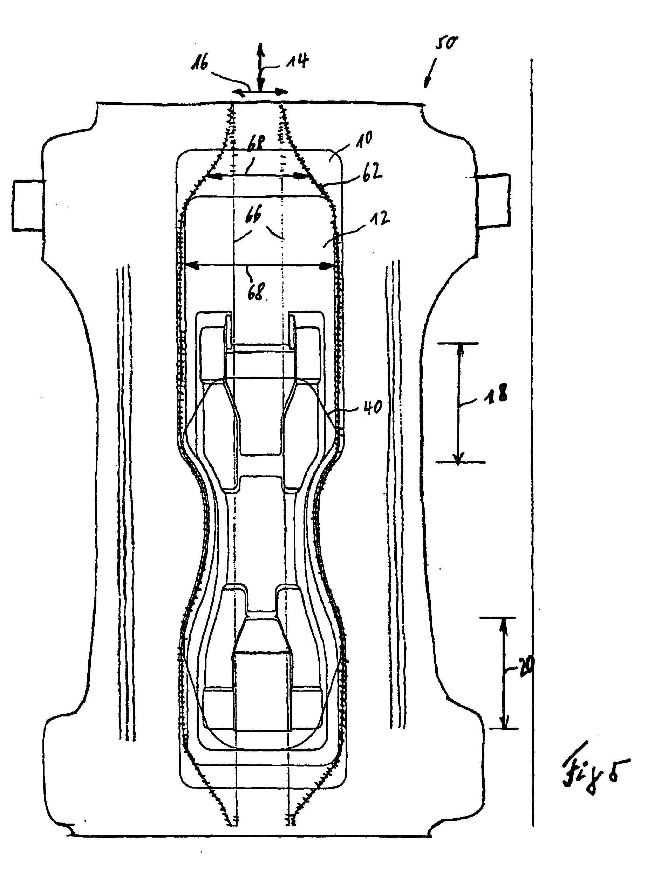

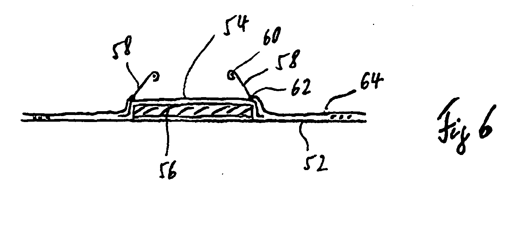

Schließlich zeigt Figur 5 eine schematische Darstellung in Draufsicht und Figur 6 eine Schnittansicht eines erfindungsgemäßen Hygieneartikels 50 in Form einer Windel. Der Hygieneartikel 50 umfasst ein flüssigkeitsundurchlässiges körperabgewandt angeordnetes Backsheet 52 und ein flüssigkeitsdurchlässiges Topsheet 54 mit einem dazwischen angeordneten Saugkörper 56, der die vorausgehend beschriebene Grundmatte 10, die Saugkörperkomponente 12 und die Verteilerschicht 40 umfasst. In der schematischen Darstellung der Figur 5 ist die Topographie der Saugkörperkomponente 12 nach Figur 2 ebenfalls ersichtlich. Ferner sind seitliche Flüssigkeitsbarrieren, sogenannte aufstehende Cuffelemente 58 mit Elastifizierungselementen 60 am distalen Ende angedeutet. Diese an sich bekannten Cuffelemente 58 sind entlang einer Cuffsockellinie 62 mit den Chassismaterialien, üblicherweise mit einer Vlieskomponente des Topsheets 54 und/oder mit einer weiteren Deckschichtkomponente 64 verbunden. Das Bezugszeichen 66 deutet in Figur 5 das distale Ende der Cuffelemente 58 an. Man erkennt aus der Darstellung in Figur 5 einen über die Längsrichtung 14 variierenden Abstand 68 der Cuffsockellinien 62 voneinander, und zwar in Querrichtung 16 des Hygieneartikels 50. Man erkennt, dass der Abstand der Cuffsockellinien über die Längserstreckung des Hygieneartikels zwei Maxima durchläuft, und dass sich die Maxima mit dem jeweiligen ersten Bereich 18, 20 zunehmenden Flächengewichts einer absorbierenden Komponente in Querrichtung 16 überlappen. Auf diese Weise ist sichergestellt, dass in Bereichen maximalen Abstands 68 der Cuffsockellinien 62 voneinander eine hohe Absorptionskapazität im Saugkörperrandbereich zur Verfügung gestellt ist. Diese ersten Bereiche 18, 20 zunehmenden Flächengewichts in Querrichtung sind etwa streifenförmig in Seitenrandbereichen des Saugkörpers 56 erstreckt. Sie bilden in der Draufsicht etwa die Form der Schenkel eines Buchstabens "H" oder "X", wobei im dargestellten Fall im Schrittbereich oder zumindest an das Zentrum des Schrittbereichs angrenzend keine Zunahme des Flächengewichts eines absorbierenden Materials in Querrichtung des Hygieneartikels vorgesehen ist.

**Patentansprüche**

1. Hygieneartikel zum einmaligen Gebrauch, mit einer der Speicherung von Körperflüssigkeiten dienenden Saugkörperkomponente (12), die auch superabsorbierende Materialien enthalten kann, wobei die Saugkörperkomponente (12) einen ersten Bereich (18, 20) mit in Querrichtung (16) des Hygieneartikels zu den Seitenrändern hin zunehmendem Flächengewicht eines absorbierenden Materials dieser Saugkörperkomponente (12) aufweist, und wobei die Saugkörperkomponente (12) zudem einen zweiten Bereich (32) mit ausgehend vom Rücken- (22) und Vorderbereich (24) des Hygieneartikels zum Schrittbereich (26) hin zunehmendem Flächengewicht eines absorbierenden Materials der Saugkörperkomponente (12) aufweist und wobei die Spelcherkapazität eines sich in Längsrichtung (14) über 40-90% der Länge der Saugkörperkomponente (12) erstreckenden Abschnitts (44) in Längsrichtung (14) betrachtet im Wesentlichen konstant ist.

2. Hygieneartikel nach Anspruch 1, **dadurch gekennzeichnet, dass** sich der Abschnitt (44) konstanter Speicherkapazität über 40 - 70%, und weiter insbesondere über 45 bis 60% der Länge der Saugkörperkomponente (12) erstreckt.

3. Hygieneartikel nach einem oder mehreren der vorstehenden Ansprüche, **dadurch gekennzeichnet, dass** der erste Bereich (18, 20) zunehmenden Flächengewichts eines absorbierenden Materials der Saugkörperkomponente (12) eine Flächengewichtszunahme in Querrichtung (16) um 30 - 200 %, insbesondere um 30 - 150 %, insbesondere 50 - 120 % aufweist.

4. Hygieneartikel nach einem oder mehreren der vorstehenden Ansprüche, **dadurch gekennzeichnet, dass** der zweite Bereich (32) zunehmenden Flächengewichts eines absorbierenden Materials der Saugkörperkomponente (12) eine Flächengewichtszunahme in Längsrichtung (14) um 50 - 500 %, insbesondere um 50 - 400 %, insbesondere 100 - 350 % aufweist.

5. Hygieneartikel nach einem oder mehreren der vorstehenden Ansprüche, **dadurch gekennzeichnet; dass** das maximale Flächengewicht des zweiten Bereiches (32) zunehmenden Flächengewichts größer ist als das maximale Flächengewicht des ersten Bereiches (18, 20) zunehmenden Flächengewichts.

6. Hygieneartikel nach einem oder mehreren der vorstehenden Ansprüche, **dadurch gekennzeichnet, dass** der erste Bereich (18, 20) oder eine Linie maximalen Flächengewichts des ersten Bereichs (18, 20) in Längsrichtung beidseits des Hygieneartikels über wenigstens 15 % der Länge der Saugkörperkomponente (12) erstreckt ist.

7. Hygieneartikel nach Anspruch 6, **dadurch gekennzeichnet, dass** der erste Bereich (18, 20) oder eine Linie maximalen Flächengewichts des ersten Bereichs (18, 20) beidseits in Längsrichtung entlang von Seitenrandbereichen des Hygieneartikels erstreckt sind.

8. Hygieneartikel nach einem oder mehreren der vorstehenden Ansprüche, **dadurch gekennzeichnet, dass** ein erster Bereich (18, 20) zunehmenden Flächengewichts in Querrichtung (16) und ein zweiter Bereich (32) zunehmenden Flächengewichts in Längsrichtung (14) unmittelbar angrenzend aneinander oder überlappend miteinander angeordnet sind.

9. Hygieneartikel nach einem oder mehreren der vorstehenden Ansprüche, **dadurch gekennzeichnet, dass** sich die Breite der Saugkörperkomponente (12) ausgehend vom Rücken- (22) und/oder Vorderbereich (24) des Hygieneartikels zum Schrittbereich (28) hin verringert.

10. Hygieneartikel nach einem oder mehreren der vorstehenden Ansprüche, **dadurch gekennzeichnet, dass** die Saugkörperkomponente mindestens zwei Saugkörperschichten umfasst.

11. Hygieneartikel nach Anspruch 14, **dadurch gekennzeichnet, dass** eine der Saugkörperschichten ein im Wesentlichen einheitliches Flächengewicht aufweist.

**12.** Hygieneartikel nach einem oder mehreren der vorstehenden Ansprüche, **dadurch gekennzeichnet, dass** die Saugkörperkomponente eine vernetzte Zellulosefasern umfassende Saugkörperschicht (40) aufweist.

**13.** Hygieneartikel nach einem oder mehreren der vorstehenden Ansprüche, **dadurch gekennzeichnet, dass** der Hygieneartikel seitliche Auslaufsperren bildende, im Wesentlichen in einer Längsrichtung (14) verlaufende zumindest bereichsweise aufstehende Cuffelemente (58), die zumindest entlang einer Cuffsockellinie (62) an der körperzugewandten Seite des Artikels festgelegt sind, aufweist.

**14.** Hygieneartikel nach Anspruch 13, **dadurch gekennzeichnet, dass** die Cuffelemente (58) mit variierendem Abstand der Cuffsockellinien, (62) voneinander geführt sind.

**15.** Hygieneartikel nach Anspruch 13 oder 14, **dadurch gekennzeichnet, dass** der erste Bereich (18, 20) größeren Flächengewichts zumindest einen Teilbereich aufweist, in dem der Abstand (66) der Cuffsockellinien (62) voneinander größer ist als außerhalb dieses Teilbereichs.

**16.** Hygieneartikel nach Anspruch 13, 14, oder 15, **dadurch gekennzeichnet, dass** die Cuffelemente (58) so geführt sind, dass der Abstand (66) der Cuffsockellinien (62) voneinander in Längsrichtung (14) ein Maximum durchläuft.

**17.** Hygieneartikel nach Anspruch 16, **dadurch gekennzeichnet, dass** das Maximum vollständig innerhalb eines Teilbereichs des Bereichs (18, 20) größeren Flächengewichts liegt.

**18.** Hygieneartikel nach Anspruch 16 oder 17, **dadurch gekennzeichnet, dass** das Maximum oder die Maxima des Abstands (66) der Cuffsockellinien (62) voneinander in einem Vorderbereich (24) und/oder in einem Rückenbereich (22) des Hygieneartikels angeordnet ist oder sind.

**19.** Hygieneartikel nach einem oder mehreren der vorstehenden Ansprüche, **dadurch gekennzeichnet, dass** die Saugkörperkomponente (12) eine Mischung aus Fasern und partikelförmigen superabsorbierenden Materialien umfasst.

## Claims

**1.** Disposable hygiene article, comprising an absorbing element component (12) for storing body liquids, which may also contain superabsorbent materials, wherein the absorbing element component (12) comprises a first area (18, 20) with a mass per unit area of an absorbent material of this absorbing element component (12) that increases in the transverse direction (16) of the hygiene article towards the side edges, and wherein the absorbing element component (12) also comprises a second area (32) with a mass per unit area of an absorbent material of the absorbing element component (12), which increases from the rear (22) and front area (24) of the hygiene article towards the crotch area (26) and wherein the storage capacity of a section (44) extending in the longitudinal direction (14) over 40 to 90 % of the length of the absorbing element component (12) is substantially constant as viewed in the longitudinal direction (14).

**2.** Hygiene article according to claim 1, **characterized in that** the section (44) of constant storage capacity extends over 40 to 70 %, and preferentially 45 to 60 % of the length of the absorbing element component (12).

**3.** Hygiene article according to any one or more of the preceding claims, **characterized in that** the first area (18, 20) of increasing mass per unit area of an absorbent material of the absorbing element component (12) increases in the transverse direction (16) by 30 to 200 %, in particular 30 to 150 %, preferentially 50 to 120 %.

**4.** Hygiene article according to any one or more of the preceding claims, **characterized in that** the second area (32) of increasing mass per unit area of an absorbent material of the absorbing element component (12) has a mass per unit area increase in the longitudinal direction (14) of 50 to 500 %, in particular 50 to 400 %, preferentially 100 to 350 %.

**5.** Hygiene article according to any one or more of the preceding claims, **characterized in that** the maximum mass per unit area of the second area (32) of increasing mass per unit area is larger than the maximum mass per unit area of the first area (18, 20) of increasing mass per unit area.

6. Hygiene article according to any one or more of the 6preceding claims, **characterized in that** the first area (18, 20) or a line of maximum mass per unit area of the first area (18, 20) extends in the longitudinal direction on both sides of the hygiene article over at least 15 % of the length of the absorbing element component (12).

7. Hygiene article according to claim 6, **characterized in that** the first area (18, 20) or a line of maximum mass per unit area of the first area (18, 20) extends in the longitudinal direction on both sides along side edge areas of the hygiene article.

8. Hygiene article according to any one or more of the preceding claims, **characterized in that** a first area (18, 20) of increasing mass per unit area in a transverse direction (16) and a second area (32) of increasing mass per unit area in a longitudinal direction (14) are disposed in direct abutment to or overlapping each other.

9. Hygiene article according to any one or more of the preceding claims, **characterized in that** the width of the absorbing element component (12) starting from the rear (22) and/or front area (24) of the hygiene article is reduced towards the crotch area (28).

10. Hygiene article according to any one or more of the preceding claims, **characterized in that** the absorbing element component comprises at least two absorbing element layers.

11. Hygiene article according to claim 10, **characterized in that** one of the absorbing element layers has a substantially uniform mass per unit area.

12. Hygiene article according to any one or more of the preceding claims, **characterized in that** the absorbing element component has an absorbing element layer (40) comprising intra-cross-linked cellulose fibers.

13. Hygiene article according to any one or more of the preceding claims, **characterized in that** the hygiene article has cuff elements (58) extending substantially in a longitudinal direction (14) and standing up at least in certain areas, which form lateral outlet barriers, and are fixed at least along a cuff bottom line (62) on the side of the article facing the body.

14. Hygiene article according to claim 13, **characterized in that** the cuff elements (58) are guided at varying separations between the cuff bottom lines (62) from each other.

15. Hygiene article according to claim 13 or 14, **characterized in that** the first area (18, 20) of larger mass per unit area comprises at least one partial area, where the mutual separation (66) between the cuff bottom lines (62) from each other is larger than outside of this partial area.

16. Hygiene article according to claims 13, 14 or 15, **characterized in that** the cuff elements (58) are guided such that the separation (66) of the cuff bottom lines (62) from each other in the longitudinal direction (14) passes through a maximum.

17. Hygiene article according to claim 16, **characterized in that** the maximum is entirely within a partial area of the area (18, 20) of larger mass per unit area.

18. Hygiene article according to claim 16 or 17, **characterized in that** the maximum or maxima of the separation (66) between the cuff bottom lines (62) from each other is/are disposed in a front area (24) and/or rear area (22) of the hygiene article.

19. Hygiene article according to any one or more of the preceding claims, **characterized in that** the absorbing element component (12) comprises a mixture of fibers and particulate superabsorbent materials.

**Revendications**

1. Article d'hygiène à usage unique comprenant un composant de corps absorbant (12) servant à retenir des fluides corporels et pouvant également contenir des matériaux superabsorbants, le composant de corps absorbant (12) présentant une première zone (18, 20) dont le poids par unité de surface d'un matériau absorbant de ce composant de corps absorbant augmente dans le sens transversal (16) de l'article d'hygiène vers les bords latéraux, le composant de corps absorbant (12) présentant en outre une deuxième zone (32) avec un poids par unité de surface d'un matériau absorbant du composant de corps absorbant (12) qui augmente depuis la zone arrière (22) et la zone avant (24) de l'article d'hygiène en direction de la zone d'entrejambe (26), et la capacité de rétention d'un segment (44) s'étendant dans le sens longitudinal (14) sur 40 à 90 % de la longueur du composant de corps absorbant (12), vue dans le sens longitudinal (14), étant essentiellement constante.

**2.** Article d'hygiène selon la revendication 1, **caractérisé en ce que** le segment (44) de capacité de rétention constante s'étend sur 40 - 70 %, et plus particulièrement sur 45 à 60 % de la longueur du composant de corps absorbant (12).

**3.** Article d'hygiène selon une ou plusieurs des revendications précédentes, **caractérisé en ce que** la première zone (18, 20) de poids par unité de surface croissant d'un matériau absorbant du composant de corps absorbant (12) présente une augmentation du poids par unité de surface dans le sens transversal (16) de 30 à 200 %, en particulier de 30 à 150 %, en particulier de 50 à 120 %.

**4.** Article d'hygiène selon une ou plusieurs des revendications précédentes, **caractérisé en ce que** la deuxième zone (32) avec un poids par unité de surface croissant d'un matériau absorbant du composant de corps absorbant (12) présente une augmentation du poids par unité de surface dans le sens longitudinal (14) de 50 à 500 %, en particulier de 50 à 400 %, en particulier de 100 à 350 %.

**5.** Article d'hygiène selon une ou plusieurs des revendications précédentes, **caractérisé en ce que** le poids par unité de surface maximal de la deuxième zone (32) de poids par unité de surface croissant est supérieur au poids par unité de surface maximal de la première zone (18, 20) de poids par unité de surface croissant.

**6.** Article d'hygiène selon une ou plusieurs des revendications précédentes, **caractérisé en ce que** la première zone (18, 20) ou une ligne du poids par unité de surface maximal de la première zone (18, 20) s'étend dans le sens longitudinal de chaque côté de l'article d'hygiène sur au moins 15 % de la longueur du composant de corps absorbant (12).

**7.** Article d'hygiène selon la revendication 6, **caractérisé en ce que** la première zone (18, 20) ou une ligne du poids par unité de surface maximal de la première zone (18, 20) s'étend de chaque côté dans le sens longitudinal le long des zones de bord latéral de l'article d'hygiène.

**8.** Article d'hygiène selon une ou plusieurs des revendications précédentes, **caractérisé en ce qu'**une première zone (18, 20) de poids par unité de surface croissant dans le sens transversal (16) et une deuxième zone (32) de poids par unité de surface croissant dans le sens longitudinal (14) sont agencées directement adjacentes ou bien en se chevauchant.

**9.** Article d'hygiène selon une ou plusieurs des revendications précédentes, **caractérisé en ce que** la largeur du composant de corps absorbant (12) diminue depuis la zone arrière (22) et/ou la zone avant (24) de l'article d'hygiène en direction de la zone d'entrejambe (28).

**10.** Article d'hygiène selon une ou plusieurs des revendications précédentes, **caractérisé en ce que** le composant de corps absorbant comprend au moins deux couches de corps absorbant.

**11.** Article d'hygiène selon la revendication 10, **caractérisé en ce que** l'une des couches de corps absorbant présente un poids par unité de surface essentiellement uniforme.

**12.** Article d'hygiène selon une ou plusieurs des revendications précédentes, **caractérisé en ce que** le composant de corps absorbant présente une couche de corps absorbant (40) comprenant des fibres de cellulose réticulées.

**13.** Article d'hygiène selon une ou plusieurs des revendications précédentes, **caractérisé en ce que** l'article d'hygiène présente des éléments de revers (58) en saillie au moins par endroits, s'étendant essentiellement dans un sens longitudinal (14) et formant des barrières latérales, lesquels éléments sont fixés au moins le long d'une ligne de base de revers (62) sur le côté de l'article d'hygiène orienté vers le corps.

**14.** Article d'hygiène selon la revendication 13, **caractérisé en ce que** les éléments de revers (58) sont guidés à une distance variable des lignes de base de revers (62) entre elles.

**15.** Article d'hygiène selon la revendication 13 ou 14, **caractérisé en ce que** la première zone (18, 20) où le poids par unité de surface est augmenté présente au moins une zone partielle, dans laquelle la distance (68) entre les lignes de base de revers (62) est supérieure par rapport à la distance en-dehors de cette zone partielle.

**16.** Article d'hygiène selon la revendication 13, 14 ou 15, **caractérisé en ce que** les éléments de revers (58) sont guidés de telle façon que la distance (68) entre les lignes de base de revers (62) parcourt un maximum dans le sens longitudinal (14).

**17.** Article d'hygiène selon la revendication 16, **caractérisé en ce que** le maximum se situe complètement à l'intérieur d'une zone partielle de la zone (18, 20) présentant une augmentation du poids par unité de surface.

**18.** Article d'hygiène selon la revendication 16 ou 17, **caractérisé en ce que** le maximum ou les maxima de la distance (68) des lignes de base de revers (62) entre elles se situent dans une zone avant (24) et/ou une zone arrière (22) de l'article d'hygiène.

**19.** Article d'hygiène selon une ou plusieurs des revendications précédentes, **caractérisé en ce que** le composant de corps absorbant (12) comprend un mélange de fibres et de matériaux superabsorbants sous forme de particules.

Sektor 21
Sektor 20
Sektor 19
Sektor 18
Sektor 17
Sektor 16
Sektor 15
Sektor 14
Sektor 13
Sektor 12
Sektor 11
Sektor 10
Sektor 09
Sektor 08
Sektor 07
Sektor 06
Sektor 05
Sektor 04
Sektor 03
Sektor 02
Sektor 01

Fig 2

Fig. 3

| RetSK | |
|---|---|
| Sektor 1 | 3 |
| Sektor 2 | 17 |
| Sektor 3 | 23 |
| Sektor 4 | 24 |
| Sektor 5 | 24 |
| Sektor 6 | 24 |
| Sektor 7 | 25 |
| Sektor 8 | 23 |
| Sektor 9 | 23 |
| Sektor 10 | 23 |
| Sektor 11 | 24 |
| Sektor 12 | 24 |
| Sektor 13 | 24 |
| Sektor 14 | 24 |
| Sektor 15 | 21 |
| Sektor 16 | 17 |
| Sektor 17 | 14 |
| Sektor 18 | 13 |
| Sektor 19 | 13 |
| Sektor 20 | 6 |
| Sektor 21 | 0 |

Fig. 4

| Retges | |
|---|---|
| Sektor 1 | 4 |
| Sektor 2 | 17 |
| Sektor 3 | 23 |
| Sektor 4 | 24 |
| Sektor 5 | 25 |
| Sektor 6 | 25 |
| Sektor 7 | 26 |
| Sektor 8 | 24 |
| Sektor 9 | 24 |
| Sektor 10 | 24 |
| Sektor 11 | 24 |
| Sektor 12 | 25 |
| Sektor 13 | 25 |
| Sektor 14 | 24 |
| Sektor 15 | 21 |
| Sektor 16 | 18 |
| Sektor 17 | 14 |
| Sektor 18 | 13 |
| Sektor 19 | 13 |
| Sektor 20 | 7 |
| Sektor 21 | 0 |

Fig 5

Fig 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10326022